# EUROPEAN PATENT SPECIFICATION

(11) **EP 1 781 852 B1**
(45) Date of publication and mention of the grant of the patent: **02.01.2013**
(21) Application number: 05782759.4
(22) Date of filing: 03.08.2005
(51) Int. Cl.: D04H 1/00, B32B 7/14, B32B 27/12, D04H 3/16, B32B 5/06, B31B 1/60

(54) **BREATHABLE ELASTIC COMPOSITE**
ATMUNGSAKTIVER ELASTISCHER VERBUNDKÖRPER
COMPOSITE ELASTIQUE PERMEABLE A L'AIR

(30) Priority: 03.08.2004 US 598319 P
(43) Date of publication of application: 09.05.2007
(73) Proprietor: Dow Global Technologies LLC, Midland, MI 48674 (US)
(72) Inventor: ABED, Jean-Claude, Simpsonville, SC 29680 (US); ROETTGER, Henning, 38108 Braunschweig (DE); WEBB, Steven,P., Midland, Michigan 48640 (US); AUSTIN, Jared, A., Greer, SC 29650 (US)
(74) Representative: Maxton Langmaack & Partner
(86) International application number: PCT/US2005/027445
(87) International publication number: WO 2006/017518

(56) References cited:
- US-A- 4 692 368
- US-A- 4 935 287
- US-A- 5 393 599
- US-A- 5 431 991
- US-A- 5 529 830
- US-A1- 2003 028 165
- US-B1- 6 680 265

## Description

This application claims priority to US provisional patent application serial number 60/598,319, filed August 3, 2004.

### BACKGROUND OF INVENTION

Laminates of breathable films and nonwoven materials are commonly used as moisture permeable liquid barriers providing a good touch. Typical applications are diaper (backsheets, side panels, and ear components), protective apparel medical gowns and drapes. Different technologies are established to produce such laminates. For example, breathable films, which may be monolithic or microporous films, are laminated with standard non-elastic nonwoven materials using bonding technologies like hot-melt adhesive lamination and thermo-bonding. Another example is a non-elastic nonwoven that is extrusion coated with a monolithic breathable polymer. Moisture is transported across such monolithic films via a solution/diffusion process and not across open voids which results in a lack of air permeability. Another example is a non-elastic nonwoven that is laminated to a non-elastic film containing inorganic fillers and subsequently stretched by means like incremental stretching/ring-rolling or tenter frames resulting in micro-voids next to the inorganic filler. These micro-voids provide moisture-permeability and air-permeability to the laminate. Another example is a non-elastic nonwoven that is extrusion coated with a polymer including an inorganic filler like calcium carbonate. The resulting composite is stretched by means such as incremental stretching/ringrolling or tenter frames resulting a micro-voids next to the inorganic filler. These micro-voids provide moisture- and air-permeability to the laminate. US 5,865,926 discloses a process for producing such a breathable laminate.

All film/nonwoven laminates mentioned above are breathable but not elastic due to the nature of the film and nonwoven materials used. Therefore they do not meet the requirement for improved body fit developing in the market. To date the only execution for an elastic breathable film/nonwoven laminate is the use of an elastic breathable film laminated to standard nonwoven materials. Such films require specific resin design and are significantly more expensive than the breathable films produced by activation of non-elastic films with inorganic fillers. Furthermore such monolithic elastic films don't provide air permeability and the same level of moisture permeability.

### SUMMARY OF INVENTION

The inventors have now recognized that a need exists for a breathable elastic composite formed from a relatively inexpensive non-elastic film layer and from an inexpensive elastic nonwoven layer. The present invention thus provides a solution to one or more of the disadvantages and deficiencies described above.

In general, the present invention provides an elastic multilayer composite as claimed in claim 1. In one embodiment, the elastic nonwoven (e.g., the spunbonded fabric) is laminated to a film that contains inorganic fillers or an immiscible phase. The film is non-elastic. The laminate is then stretched (incremental or integral) and released to provide a breathable structure by the generation of micro voids. In another embodiment, the elastic nonwoven is extrusion coated with a polymeric film that contains inorganic fillers or an immiscible phase. The laminate is then stretched (incremental or integral) and released to provide a breathable structure by the generation of micro voids. Likewise, in another embodiment the elastic nonwoven can be stretched prior to lamination against a breathable non-elastic film. After relaxation of the laminate the film is bulked/gathered by the retractive force of the elastic nonwoven.

This present invention describes a product comprised of breathable non-elastic film and an elastic spunbonded fabric layer. The non-elastic film layer can be breathable prior to forming the composite, or can be made breathable as by stretching subsequent to forming the composite.

Advantageously, the current invention provides for a truly elastic breathable film/nonwoven composite using established and cost efficient breathable film technology. The use of microporous films allows the production of air-permeable and moisture-permeable elastic film nonwoven composites which are not achievable with current breathable elastic films. Compared to the combination of a breathable elastic film and a non-elastic nonwoven which needs to be activated in order to achieve an elastic laminate, this invention provides the benefit that the elastic nonwoven is unharmed by mechanical stretching and retains, or improves, its inherent properties such as abrasion resistance, tensile properties, tactile properties, and elastic properties.

In one broad respect, this invention is a breathable elastic multilayer composite, comprising a breathable non-elastic film layer and an elastic nonwoven layer. The elastic nonwoven layer can be a spunbonded fabric.

In another broad respect, this invention is a process for manufacturing a elastic multilayer composite as claimed in claim 14.

The non-elastic film can be in the form of a multilayered film, a monolithic film, a cast film.

As used herein, the elastic nonwoven may be formed by any nonwoven process. Preferably the nonwoven is an elastic spunbonded nonwoven. The elastic spunbonded nonwoven may be made up of bicomponent fibers. The bicomponent elastic spunbond can be produced in a manner such that bonding of the fibers occurs significantly only during a standard thermobonding step with a heated calendar roll or in a standard adhesive process. The invention can be practiced in the absence of a so-called stabilization step sometimes employed in the art, such as use of a hot air knife over the composite. Preferably, a heated press roll (compaction roll, as is well known in the art), but at a temperature below which significant bonding might occur (i.e. below the bonding temperature that may be used to thermopoint bond the web), may be used. In one embodiment, any post-web processing or treatment, prior to web point bonding, is done at a temperature/pressure low enough so that extensive inter-fiber bonding does not occur.

### BRIEF DESCRIPTION OF THE DRAWINGS

Figure 1 shows an extrusion lamination process that may be used in the practice of this invention.
Figure 2 shows a melt adhesive lamination process that may be used in the practice of this invention.
Figure 3 shows an adhesive lamination process that may be used in the practice of this invention.

### DETAILED DESCRIPTION OF THE INVENTION

While additional layers can be added to the composite of this invention, the basic structure of the composite can be referred to as an A-B structure where "A" is an elastic nonwoven layer and "B" is a breathable non-elastic film layer. Alternatively, the composite can have an A-B-A or B-A-B structure, or other multilayer composite structure, including structure that have non-A or non-B layers (excluding adhesive layers). It should be understood that an adhesive may be employed to laminate the A and B layers together. Likewise, multilayer composites having more than three layers are within the scope of this invention, including composites made of one or more layers other than A and B.

The film-nonwoven composite could be produced by extrusion lamination of the film onto an elastic nonwoven or by adhesive lamination to/between one or more elastic nonwovens. Alternatively, the composite can be manufactured by casting (direct or off-line), especially with aqueous dispersions. Another alternative method is by thermally bonding to form thermal bonded laminates, such technique being described in US 5,683,787. All of the above lamination techniques could be accomplished under neutral tension between the film and the nonwoven. Subsequent to bonding the composite, the laminate can be stretched and relaxed. Also, while the elastic nonwoven can be activated before or after lamination, activation may not be required. Thus, there would not necessarily be a need to pre-activate the elastic nonwoven prior to, or after, lamination. Over time, and multiple stretches, the overall integrity of the elastic composite is expected to be superior to that of a composite produced from an elastic film and non-elastic nonwoven. This would translate in better overall abrasion resistance, sustained nonwoven integrity, and overall general appearance.

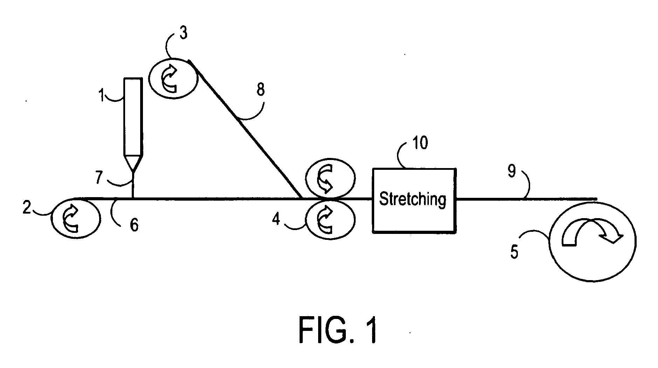

Figure 1 (referred to in the drawings as "FIG. 1 ") depicts extrusion lamination to form a composite where a non-elastic film layer is laminated to an elastic nonwoven layer. In Figure 1, a first elastic nonwoven layer 6 is unwound from unwind roll 2 or directly produced via a spunbond process. The first elastic nonwoven layer 6 comes in contact with molten non-elastic polymer 7, which is deposited via non-elastic film melt extruder 1 and which, upon cooling, forms the inner non-elastic film layer. Next, a second, optional elastic nonwoven layer 8 from second roll 3 is unwound so as to contact the inelastic film layer and thereby form a three layer mass which is laminated together via pressure nips 4. The resulting composite 9 is then stretched 10 (incrementally or integrally) to impart breathability to the film, tension is then released, and the resulting composite is then wound onto laminate rewind roll 5.

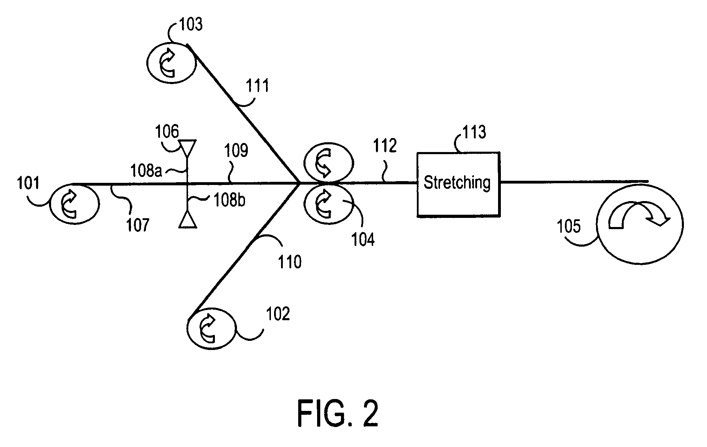

In Figure 2 (referred to in the drawings as "FIG. 2"), an adhesive lamination process is shown. A non-elastic film 107 (breathable or containing inorganic fillers or an immiscible phase) is unwound from film roll 101 and moves forward toward laminate rewind roll 105. Adhesive layers 108a and optionally 108b are applied via melt adhesive sprayers 106 to each side of the non-elastic film. The adhesive can be a hot melt adhesive. Representative, non-limiting examples of commercially available hot melt adhesives include Ato Findley H9282F, Ato Findley H2120, and HP Fuller HL-1470. The adhesive-sprayed non-elastic film 109 moves forward to pressure nip 104 where a first and an optional second elastic nonwoven layers 110 and 111 that are unwound from nonwoven rolls 102 and 103 are brought into contact with each respective side of the film 109. The layers 110 and 111 are laminated to the film 109 by the pressure from the nip 104, with the resulting composite 112 exiting the nip 104. The composite 112 is then stretched 113 (incrementally or integrally), if necessary, to impart breathability to the film and then wound onto laminate rewind roll 105.

Still referring to Figure 2, in the case of thermal lamination, a non-elastic film 107 (breathable or containing inorganic fillers or an immiscible phase) is unwound from film roll 1 and moves forward toward laminate rewind roll 105. The layers 110 and 111 are then laminated to the film 107 by the process of temperature and pressure at the nip 104, with the resulting composite 112 exiting the nip 104. The composite is then stretched 113 (incrementally or integrally), if necessary to impart breathability to the film and then wound onto laminate rewind roll 105. In the thermal lamination process, hot melt adhesive 108a, 108b is not applied to the film 107.

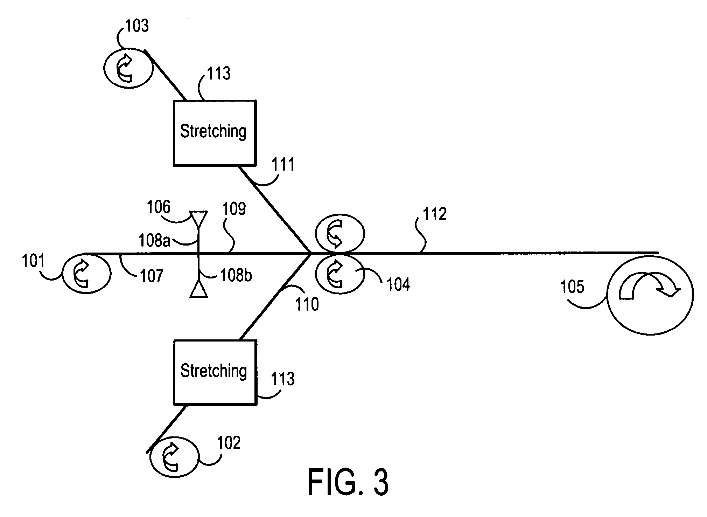

In Figure 3 (referred to in the drawings as "FIG. 3"), another adhesive lamination process is depicted. A non-elastic (breathable or containing inorganic fillers or an immiscible phase) film 107 is unwound from film roll 101 and moves forward toward laminate rewind roll 105. Adhesive layers 108a and optionally 108b are applied via melt adhesive sprayers 106 to each side of the non-elastic film. The adhesive can be a hot melt adhesive. The adhesive-sprayed non-elastic film 109 moves forward to pressure nip 104. Here it is joined with a first and an optional second elastic nonwoven layers 110 and 111 that have been unwound from nonwoven rolls 102 and 103, stretched 113 integrally in the MD, CD, or both directions and brought into contact while under tension with each respective side of the film 109. The layers 110 and 111 are laminated to the film 109 by the pressure from the nip 104, with the resulting composite 112 exiting the nip 104. Once exiting the nip the tension is released and, if necessary the composite is further stretched (incrementally or integrally) to impart breathability. The composite is then wound onto laminate rewind roll 105.

Still referring to Figure 3, in the case of thermal lamination, a breathable non-elastic film 107 is unwound from film roll 101 and moves forward toward laminate rewind roll 105. Here it is joined with a first and an optional second elastic nonwoven layers 110 and 111 that have been unwound from the nonwoven rolls 102 and 103, stretched 113, and brought into contact while under tension with each respective side of the film 109. The layers 110 and 111 are then laminated to the film 107 by the process of temperature and pressure at nip 104, with the resulting composite 112 exiting the nip 104. Once exiting the nip the tension is released and the composite is then wound onto laminate rewind roll 105. In the thermal lamination process, hot melt adhesive 108a, 108b is not applied to the film 107.

The temperatures, rate of production, selection of film, selection of adhesive, selection of elastic nonwoven, and so on can be readily selected and/or determined.

The breathable non-elastic film may comprise either a mono-layer or multilayer film. In addition, microporous films are believed suitable for use with the present invention. Breathability can be imparted by selection of materials to make the film, by being porous, by having holes formed through the film, and so on. Breathability can alternatively be imparted during the production of the composite of this invention, such as by stretch activation. The films can be made from moisture permeable or moisture impermeable materials. Some films are made breathable by adding micropore developing filler particles to the film during the film forming process. A micropore developing filler is meant to include particulates and other forms of materials which can be added to a polymer and which will not chemically interfere with or adversely affect the extruded film made from the polymer but are able to be uniformly dispersed throughout the film. Generally, the micropore developing fillers will be in particulate form and usually will have somewhat of a spherical shape with average particle sizes in the range of about 0.5 to about 8 microns. The film will usually contain at least about 30 percent of micropore developing filler based upon the total weight of the film layer. Both organic and inorganic micropore developing fillers are contemplated to be within the scope of the present invention provided that they do not interfere with the film formation process, the breathability of the resultant film or its ability to bond to a fibrous elastic nonwoven web. Examples of micropore developing fillers include calcium carbonate, various kinds of clay, silica, alumina, barium sulfate, sodium carbonate, talc, magnesium sulfate, titanium dioxide, zeolites, aluminum sulfate, cellulose-type powders, diatomaceous earth, magnesium sulfate, magnesium carbonate, barium carbonate, kaolin, mica, carbon, calcium oxide, magnesium oxide, aluminum hydroxide, glass particles, pulp powder, wood powder, cellulose derivative, polymer particles, chitin and chitin derivatives. The micropore developing filler particles may optionally be coated with a fatty acid, such as stearic acid, or a larger chain fatty acid such as behenic acid, which may facilitate the free flow of the particles (in bulk) and their ease of dispersion into the polymer matrix. Silica-containing fillers may also be present in an effective amount to provide antiblocking properties.

Once the particle-filled film has been formed, it is then either stretched or crushed to create pathways through the film. Generally, to qualify as being "breathable" for the present invention, the resultant laminate should have a water vapor transmission rate (WVTR) of at least about 250 g/m² /24 hours as may be measured by a test method as described below. Furthermore, the films may be apertured. In forming the films, the films may be coextruded to increase bonding and alleviate die lip build-up. Processes for forming films and multilayer films are generally known. The film 15 can be made from either cast or blown film equipment, can be coextruded and can be embossed if so desired. Additionally, the film 15 can be stretched or oriented by passing the film through a film stretching unit. The stretching may reduce the film gauge or thickness. Generally, this stretching may take place in the CD or MD or both. The non-elastic film may comprise a barrier layer and may also exhibit good drape. The non-elastic films may have a basis weight between about 15 grams per square meter and 100 grams per square meter, and in one embodiment between about 20 grams per square meter and 60 grams per square meter. The term "film" as used herein refers to a thin article and includes strips, tapes, and ribbons of a variety of widths, lengths, and thicknesses. The film is typically flat and has a thickness up to about 50 mils, more typically up to about 10 mils. Thermoplastic polymers used in the fabrication of the breathable non-elastic films include, but are not limited to, polyolefins including homopolymers, copolymers, terpolymers, and blends thereof. The polymers have a melt index and other properties that will produce nonelastic films. Representative examples of such non-elastomeric polyolefins include polymers of ethylene, propylene, butylene, pentene, hexene, heptene, and octene, as well as copolymers, terpolymers, and blends thereof. The non-elastomeric film may also be made with ethylene vinyl acetate (EVA), ethylene ethyl acrylate (EEA), ethylene acrylic acid (EAA), ethylene methyl acrylate (EMA), ethylene butyl acrylate, polyurethane, poly(ether-ester) and poly(amid-ether) block copolymers, and any combination thereof, including combinations with one or more polyolefins.

Nonwovens are commonly made by melt spinning thermoplastic materials. Such nonwovens are called "spunbond" or "meltblown" materials and methods for making these polymeric materials are also well known in the field. Spunbonded fabrics are employed in this invention due to advantageous economics. While spunbond materials with desirable combinations of physical properties, especially combinations of softness, strength and durability, have been produced, significant problems have been encountered. The nonwovens employed in this invention are typically conjugate fibers and typically bicomponent fibers. In one embodiment the nonwoven is made from bicomponent fibers having a sheath/core structure. Representative bicomponent, elastic nonwovens and the process for making them, suitable for this invention, are given by Austin in WO 00/08243.

Elastic nonwoven fabrics can be employed in a variety of environments such as bandaging materials, garments such as work wear and medical gowns, diapers, support clothing, incontinence products, diapers, training pants, and other personal hygiene products because of their breathability as well as their ability to allow more freedom of body movement than fabrics with more limited elasticity. Of particular relevance to this invention are articles that form diaper backsheets, protective apparel, medical gowns, and drapes.

As used herein, the term "strand" is being used as a term generic to both "fiber" and filament". In this regard, "filaments" are referring to continuous strands of material while "fibers" mean cut or discontinuous strands having a definite length. Thus, while the following discussion may use "strand" or "fiber" or "filament", the discussion can be equally applied to all three terms.

Specifically, what is about to be described hereinbelow for the elastic nonwoven are what we would define as "chemically" elastic fibers. To those skilled in the art it will be readily apparent the distinction of these fibers from the less elastic, 1-dimensionally elastic, "physical" or "mechanical" elastic nonwovens produced via heat stretching of an otherwise essentially inelastic nonwoven.

Briefly, the bicomponent strands used to make the elastic nonwoven are typically composed of a first component and a second component. The first component is an "elastic" polymer(s) which refers to a polymer that, when subjected to an extension, deforms or stretches within its elastic limit (i.e., it retracts when released). Many fiber forming thermoplastic elastomers are known in the art and include polyurethanes, block copolyesters, block copolyamides, styrenic block polymers, and polyolefin elastomers including polyolefin copolymers. Representative examples of commercially available elastomers for the first (inner) component include the KRATON polymers sold formerly by Kraton Corp.; ENGAGE elastomers (sold by Dupont Dow Elastomers), VERSIFY elastomers (produced by Dow Chemical) or, VISTAMAXX (produced by Exxon-Mobile Corp.) polyolefin elastomers; and the VECTOR polymers sold by DEXCO. Other elastomeric thermoplastic polymers include polyurethane elastomeric materials ("TPU"), such as PELLETHANE sold by Dow Chemical, ELASTOLLAN sold by BASF, ESTANE sold by B.F. Goodrich Company; polyester elastomers such as HYTREL sold by E.I. Du Pont De Nemours Company; polyetherester elastomeric materials, such as ARNITEL sold by Akzo Plastics; and polyetheramide materials, such as PEBAX sold by Elf Atochem Company. Heterophasic block copolymers, such as those sold by Montel under the trade name CATALLOY are also advantageously employed in the invention. Also suitable for the invention are polypropylene polymers and copolymers described in U.S. Pat. No. 5,594,080.

The second component is also a polymer(s), preferably a polymer which is extensible. Any thermoplastic, fiber forming, elastic polymer would be possible as the second component, depending on the application. Cost, stiffness, melt strength, spin rate, stability, etc will all be a consideration. The second component is formed from a polymer or polymer composition exhibiting inferior elastic properties in comparison to the polymer or polymer composition used to form the first component. Fiber-forming thermoplastic polymers include polyethylene (including LLDPE), polypropylene, and blends thereof. The second component polymer may have elastic recovery and may stretch within its elastic limit as the bicomponent strand is stretched. However, this second component is selected to provide poorer elastic recovery than the first component polymer. The second component may also be a polymer which can be stretched beyond its elastic limit and permanently elongated by the application of tensile stress. For example, when an elongated bicomponent filament having the second component at the surface thereof contracts, the second component will typically assume a compacted form, providing the surface of the filament with a rough appearance.

In order to have the best elastic properties, it is advantageous to have the elastic first component occupy the largest part of the filament cross section. In one embodiment, when the strands are employed in a bonded web environment, the bonded web has a root mean square average recoverable elongation of at least about 65% based on machine direction and cross direction recoverable elongation values after 50% elongation and one pull. The root mean square average recoverable elongation is the square root of the sum of (percent recovery in the machine direction)² + percent recovery in the cross machine direction)².

The second component is in an amount less than about 50 percent by weight of the strand, with between about 1 and about 20 percent in one embodiment and about 5-10 percent in another embodiment, depending on the exact polymer(s) employed as the second component.

In one respect, where the second component is substantially not elastic resulting in the strand being not elastic as a whole, in one embodiment the second component is present in an amount such that the strand becomes elastic upon stretching of the strand by an amount sufficient to irreversibly alter the length of the second component.

Suitable materials for use as the first and second components are selected based on the desired function for the strand. Preferably, the polymers used in the components of the invention have melt flows from about 5 to about 1000. Generally, the meltblowing process will employ polymers of a higher melt flow than the spunbonded process.

These bicomponent strands can be made with or without the use of processing additives. In the practice of this invention, blends of two or more polymers can be used for either the first component or second component or both.

The first component forms the majority of the fiber, i.e., greater than about 50 percent by weight, based on the weight of the strand ("bos"). For example, the first component may beneficially be present in the multicomponent strand in an amount ranging from about 80 to 99 weight percent bos, such as in an amount ranging from about 85 to 95 weight percent bos. In such advantageous embodiments, the non-elastomeric component would be present in an amount less than about 50 weight percent bos, such as in an amount of between about 1 and about 20 weight percent bos. In beneficial aspects of such advantageous embodiments, the second component may be present in an amount ranging from about 5 to 15 weight percent bos, depending on the exact polymer(s) employed as the second component. In one advantageous embodiment, a sheath/core configuration having a core to sheath weight ratio of greater than or equal to about 85:15 is provided, such as a ratio of 95:5.

The shape of the fiber can vary widely. For example, typical fiber has a circular cross-sectional shape, but sometimes fibers have different shapes, such as a trilobal shape, or a flat (i.e., "ribbon" like) shape. Also the fibers, even though of circular cross-section, may assume a non-cylindrical, 3-dimentional shape, especially when stretched and released (self-bulking or self-crimping to form helical or spring-like fibers).

For the inventive elastic fibers disclosed herein, the diameter can be widely varied. The fiber denier can be adjusted to suit the capabilities of the finished article. Expected fiber diameter values would be: from about 5 to about 20 microns/filament for melt blown; from about 10 to about 50 micron/filament for spunbond; and from about 20 to about 200 micron/filament.

Basis weight refers to the area density of a non-woven fabric, usually in terms of g/m² or oz/yd². Acceptable basis weight for a nonwoven fabric is determined by application in a product. Generally, one chooses the lowest basis weight (lowest cost) that meets the properties dictated by a given product. For elastomeric nonwovens one issue is retractive force at some elongation, or how much force the fabric can apply after relaxation at a certain extension. Another issue defining basis weight is coverage, where it is usually desirable to have a relatively opaque fabric, or if translucent, the apparent holes in the fabric should be of small size and homogeneous distribution. The most useful basis weights in the nonwovens industry for disposable products range from 1/2 to 3 oz/yd² (17 to 100 g/m², or gsm). Some applications, such as durable or semi-durable products, may be able to tolerate even higher basis weights. It should be understood that low basis weight materials may be adventitiously produced in a multiple beam construction. That is, it may be useful to produce an SMS (spunbond/meltblown/spunbond) composite fabric where each of the individual layers have basis weights even less than 17 gsm, but it is expected that the preferred final basis weight will be at least 17 gsm.

A nonwoven composition or article is typically a web or fabric having a structure of individual fibers or threads which are randomly interlaid, but not in an identifiable manner as is the case for a woven or knitted fabric.

The first and second polymeric components can optionally include, without limitation, pigments, antioxidants, stabilizers, surfactants, waxes, flow promoters, solid solvents, particulates and material added to enhance processability of the composition.

Nonwoven webs can be produced by techniques that are recognized in the art. A class of processes, known as spunbonding is the most common method for forming spunbonded webs. Examples of the various types of spunbonded processes are described in U.S. Pat. No. 3,338,992 to Kinney, U.S. Pat. No. 3,692,613 to Dorschner, U.S. Pat. No. 3,802,817 to Matsuki, U.S. Pat. No. 4,405,297 to Appel, U.S. Pat. No. 4,812,112 to Balk, and U.S. Pat. No. 5,665,300 to Brignola et al. In general, these spunbonded processes include:
a) extruding the strands from a spinneret;
b) quenching the strands with a flow of air which is generally cooled in order to hasten the solidification of the molten strands;
c) attenuating the filaments by advancing them through the quench zone with a draw tension that can be applied by either pneumatically entraining the filaments in an air stream or by wrapping them around mechanical draw rolls of the type commonly used in the textile fibers industry;
d) collecting the dawn strands into a web on a foraminous surface; and
e) bonding the web of loose strands into a fabric.

Any processing or handling of the web, between steps (d) and (e) should be done, in accordance with this invention, at a temperature below which interfiber bonding does not significantly occur.

This bonding (step (e)) can be any thermal or chemical bonding treatment, and may be used to form a plurality of intermittent bonds, such that a coherent web structure results. Thermal point bonding is most preferred. Various thermal point bonding techniques are known, with the most preferred utilizing calendar rolls with a point bonding pattern. Any pattern known in the art may be used with typical embodiments employing continuous or discontinuous patterns. Preferably, the bonds cover between 6 and 30 percent, and most preferably, 16 percent of the layer is covered. By bonding the web in accordance with these percentage ranges, the filaments are allowed to elongate throughout the full extent of stretching while the strength and integrity of the fabric can be maintained.

All of the spunbonded processes of this type or others can be used to make the elastic fabric of this invention if they are outfitted with a spinneret and extrusion system capable of producing elastic filaments, especially bicomponent filaments.

Another class of process, known as meltblowing, can also be used to produce the nonwoven fabrics of this invention. This approach to web formation is described in NRL Report 4364 "Manufacture of Superfine Organic Fibers" by V. A. Wendt, E. L. Boone, and C. D. Fluharty and in U.S. Pat. No. 3,849,241 to Buntin et al. The meltblowing process generally involves:
a.) Extruding the strands from a spinneret.
b.) Simultaneously quenching and attenuating the polymer stream immediately below the spinneret using streams of high velocity air. Generally, the strands are drawn to very small diameters by this means. However, by reducing the air volume and velocity, it is possible to produce strand with deniers similar to common textile fibers.
c.) Collecting the drawn strands into a web on a foraminous surface. Meltblown webs can be bonded by a variety of means, but often the entanglement of the filaments in the web provides sufficient tensile strength so that it can be wound into a roll.

Any meltblowing process which provides for the extrusion of bicomponent filaments such as that set forth in U.S. Pat. No. 5,290,626 can be used to practice this invention.

The composites of the present invention have utility in a variety of applications. Suitable applications include, for example, but are not limited to, disposable personal hygiene products (e.g., training pants, diapers, absorbent underpants, incontinence products, feminine hygiene items and the like); disposable garments (e.g., industrial apparel, coveralls, head coverings, underpants, pants, shirts, gloves, socks and the like); infection control/clean room products (e.g. surgical gowns and drapes, face masks, head coverings, surgical caps and hood, shoe coverings, boot slippers, wound dressings, bandages, sterilization wraps, wipers, lab coats, coverall, pants, aprons, jackets), and durable and semi-durable applications such as bedding items and sheets, furniture dust covers, apparel interliners, car covers, and sports or general wear apparel.

It should be appreciated that an elastic material or elastic-like nonwoven, as applicable to this invention, typically refers to any material having a root mean square average recoverable elongation of about 65% or more based on machine direction and cross-direction recoverable elongation values after 50% elongation of the web and one pull. The extent that a material does not return to its original dimensions after being stretched and immediately released is its percent permanent set. According to ASTM testing methods, set and recovery will add to 100%. Set is defined as the residual relaxed length after an extension divided by the length of extension (elongation). For example, a one inch gauge (length) sample, pulled to 200% elongation (two additional inches of extension from the original one inch gauge) and released might a) not retract at all so that the sample is now three inches long and will have 100% set ((3"_{end} - 1 "ᵢₙᵢₜᵢₐₗ)/2"ₑₓₜₑₙₛᵢₒₙ), or b) retract completely to the original one inch gauge and will have 0% set ((1"_{end} - 1 "ᵢₙᵢₜᵢₐₗ)/2"ₑₓₜₑₙₜᵢₒₙ), or c) will do something in between. An often used and practical method of measuring set is to observe the residual strain (recovery) on a sample when the restoring force or load reaches zero after it is released from an extension. This method and the above method will only produce the same result when a sample is extended 100%. For example, as in the case above, if the sample did not retract at all after 200% elongation, the residual strain at zero load upon release would be 200%. Clearly in this case set and recover will not add to 100%.

By contrast, a non-elastic film does not meet these criteria. Specifically, a non-elastic film would be expected to demonstrate less than 50%, more likely less than 25%, recovery when extended to 50% of its original length. Moreover, non-elastic films are typically described by a tensile curve that shows extensive yielding prior to break. In this regard the film will show a rapid increase in stress at small extensions followed by a near maximum, approximately constant stress at the yield point and during continued extension until the film ruptures. Prior to rupture, any release of the sample results in a mostly extensively-elongated, non-retracted film.

The elastic nonwoven and non-elastic film can be separately subjected to activation. The elastic nonwoven may be activated to thereby reduce its tensile strength and/or improve its elastic properties prior to lamination. The nonelastic film may be activated to impart breathability. Alternatively, the composite itself can be activated, in such a case, the nonwoven or film may be, but need not be, activated prior to lamination. Activation can be conducted by well known techniques. In one embodiment, if activation is desired, the nonwoven is activated so that that its tensile strength is lessened, generally lessened so that the tensile strength is below that of the film (whether or not the nonwoven has a tensile strength below that of the film prior to activation). Activation may be conducted by an initial drawing or stretching process. Traditional stretching equipment associated with wide web products include conventional draw rolls and tenter frames. The activation process may be accomplished by any drawing or stretching process known in the art, including incremental stretching, tentering, roll drawing, and the like. The activation process is generally performed after the strands have been formed into a nonwoven web or fabric, although it may be done before. The activation process generally stretches the nonwoven web or fabric about 1.1 to 10.0 fold. In advantageous embodiments, the fibers or fabric are stretched or drawn to at least about 2.5 times its initial length. The incremental stretching step may include incrementally stretching the web in both the machine direction and the cross-machine direction. Advantageously, incremental stretching may be accomplished by directing the web through at least one pair of interdigitating stretching rollers. WO 2004/038085 discloses this type of process. In one aspect of such embodiments, the interdigitating stretching rollers give rise to narrow, spaced apart longitudinally extending stretch-activated elastic zones within the fabric, separated by intervening longitudinally extending non-activated zones that are substantially less elastic. The incremental stretching may be accomplished by directing an incrementally stretched web through a second pair of interdigitating stretching rollers to stretch activate a second portion of the non-activated strands within the web. Mechanical incremental stretching may be performed in conjunction with an impinging fluid (e.g., air or water) directed onto the surface of the web.

Further modifications and alternative embodiments of this invention will be apparent to those skilled in the art in view of this description. Accordingly, this description is to be construed as illustrative only and is for the purpose of teaching those skilled in the art the manner of carrying out the invention. It is to be understood that the forms of the invention herein shown and described are to be taken as illustrative embodiments. Equivalent elements or materials may be substituted for those illustrated and described herein, and certain features of the invention may be utilized independently of the use of other features, all as would be apparent to one skilled in the art after having the benefit of this description of the invention.

## Claims

1. An elastic multilayer composite, comprising a breathable non-elastic film layer adjacent to an elastic nonwoven layer, wherein the elastic nonwoven layer is formed of bicomponent fibers, wherein the bicomponent fibers having a sheath/core multilobal, or tipped multilobal structure. Include an inner first component and an outer second component, wherein the second component is less elastic than the first component, wherein the first component is a thermoplastic elastomer, wherein the first component comprises at least 50% of the fibers, and wherein the second component is polyethylene, polypropylene, or a blend of polyethylene and polypropylene.

2. The composite of claim 1, wherein adhesive is between the breathable non- elastic film layer and the elastic nonwoven layer.

3. The composite of any of the preceding claims, wherein the layers adhere to one another at a multiplicity of points, and wherein those bond points are formed via heat and pressure.

4. The composite of any of the preceding claims, wherein the elastic nonwoven layer is a spunbonded fabric.

5. The composite of any of the preceding claims, wherein the nonwoven layer is composed of bicomponent fibers which have not been activated.

6. The composite of any of the preceding claims, wherein the nonwoven layer is composed of bicomponent fibers which have been stretch activated.

7. The composite of any of the preceding claims, wherein the composite has a two-layer structure.

8. The composite of any of claims 1 - 6, wherein the composite has a three-layer structure.

9. The composite of any of the preceding claims, wherein the entire composite is stretch activated.

10. The composite of any of the preceding claims, wherein the nonelastic film is formed of a polymer of ethylene, propylene, butylene, pentene, hexene, heptene, and octene, as well as copolymers, terpolymers, and blends thereof, ethylene vinyl acetate (EVA), ethylene ethyl acrylate (EEA), ethylene acrylic acid (EAA), ethylene methyl acrylate (EMA), ethylene butyl acrylate, polyurethane, poly(ether-ester) and poly(amid-ether) block copolymers, and any combination thereof.

11. The composite of any of the preceding claims, wherein the elastic nonwoven layer is formed of bicomponent fibers, wherein the bicomponent fibers include an inner first component and an outer second component, wherein the second component is less elastic than the first component, wherein the first component is a thermoplastic elastomer, wherein the second component is present in the bicomponent fibers in between about 1 and about 20 percent.

12. The composite of any of the preceding claims, wherein the elastic nonwoven layer is formed of bicomponent fibers, wherein the bicomponent fibers include an inner first component and an outer second component, wherein the second component is less elastic than the first component, wherein the first component is a thermoplastic elastomer, wherein the first component is formed from a polyurethane, a block copolyester, a block copolyamide, a styrenic block polymer, a polyolefin elastomer, and combinations thereof.

13. The composite of any of the preceding claims, wherein the breathable non- elastic film is a multilayered film, a monolithic film, a cast film.

14. A process for manufacturing an elastic multilayer composite, comprising: forming a composite of at least a breathable non-elastic film layer and at least one elastic nonwoven layer, wherein the elastic nonwoven layer is formed of bicomponent fibers, wherein the bicomponent fibers having a sheath/core, multilobal, or tipped multilobal structure include an inner first component and an outer second component, wherein the second component is less elastic than the first component, wherein the first component is a thermoplastic elastomer, wherein the first component comprises at least 50% of the fibers, and wherein the second component is polyethylene, polypropylene, or a blend of polyethylene and polypropylene..

15. The process of claim 14, wherein the laminate is formed using thermopoint bonding.

16. The process of claim 14, wherein the layers are formed using extrusion lamination.

17. The process of claim 14, wherein adhesive is between the breathable non- elastic film layer and the elastic nonwoven layer.

18. The process of any of claims 14-17, wherein the elastic nonwoven is a spunbonded nonwoven.

19. The process of any of claims 14-18, wherein the nonwoven layer is composed of bicomponent fibers which have not been activated.

20. The process of any of claims 14-19, wherein the nonwoven layer is composed of bicomponent fibers which have been stretch activated.

21. The process of any of claims 14-20, wherein the composite has a two-layer structure, not inclusive of an optional adhesive layer.

22. The process of any of claims 14-21, wherein the composite has a three-layer structure, not inclusive of two optional adhesive layers.

23. The process of any of claims 14-22, wherein the entire composite is stretch activated.

24. The process of any of claims 14-23, wherein the lamination is conducted using melt adhesive lamination.

25. The process of any of claims 14-24, wherein the lamination is conducted using extrusion lamination.

26. The process of any of claims 14-25, wherein the lamination is conducted at a plurality of spaced apart points using thermopoint bonding.

27. The composite of any of claims 14-26, wherein the elastic nonwoven layer has a tensile strength less than the tensile strength of the non-elastic film.

28. The composite of any of claims 14-27, wherein the nonelastic film is formed of a polymer of ethylene, propylene, butylene, pentene, hexene, heptene, and octene, as well as copolymers, terpolymers, and blends thereof, ethylene vinyl acetate (EVA), ethylene ethyl acrylate (EEA), ethylene acrylic acid (EAA), ethylene methyl acrylate (EMA)1 ethylene butyl acrylate, polyurethane, poly(ether-ester) and poly(amid-ether) block copolymers, and any combination thereof.

29. The composite of any of claims 14-28, wherein the elastic nonwoven layer is formed of bicomponent fibers, wherein the bicomponent fibers include an inner first component and an outer second component, wherein the second component is less elastic than the first component, wherein the first component is a thermoplastic elastomer, wherein the second component is present in the bicomponent fibers in between about 1 and about 20 percent.

30. The composite of any of claims 14-29, wherein the elastic nonwoven layer is formed of bicomponent fibers, wherein the bicomponent fibers include an inner first component and an outer second component, wherein the second component is less elastic than the first component, wherein the first component is a thermoplastic elastomer, wherein the first component is formed from a polyurethane, a block copolyester, a block copolyamide, a styrenic block polymer, a polyolefin elastomer, and combinations thereof.

## Patentansprüche

1. Elastischer mehrlagiger Verbundstoff, der eine atmungsaktive, nicht-elastische Folienschicht angrenzend an eine elastische Vliesschicht umfasst, wobei die elastische Vliesschicht aus Bikomponentenfasern gebildet ist, wobei die Bikomponentenfasern, die eine Kern/Mantel-, eine mehrlappige oder eine spitz mehrlappige Struktur aufweisen, eine innere erste Komponente und eine äußere zweite Komponente umfassen, wobei die zweite Komponente weniger elastisch als die erste Komponente ist, wobei die erste Komponente ein thermoplastisches Elastomer ist, wobei die erste Komponente mindestens 50 % der Fasern ausmacht und wobei die zweite Komponente Polyethylen, Polypropylen oder eine Mischung aus Polyethylen und Polypropylen ist.

2. Verbundstoff nach Anspruch 1, wobei sich Klebstoff zwischen der atmungsaktiven, nicht-elastischen Folienschicht und der elastischen Vliesschicht befindet.

3. Verbundstoff nach einem der vorhergehenden Ansprüche, wobei die Schichten an einer Vielzahl von Punkten aneinander haften, und wobei diese Verbindungspunkte mit Hilfe von Wärme und Druck erzeugt sind.

4. Verbundstoff nach einem der vorhergehenden Ansprüche, wobei die elastische Vliesschicht ein Spinnvlies ist.

5. Verbundstoff nach einem der vorhergehenden Ansprüche, wobei die Vliesschicht aus Bikomponentenfasern besteht, die nicht aktiviert wurden.

6. Verbundstoff nach einem der vorhergehenden Ansprüche, wobei die Vliesschicht aus Bikomponentenfasern besteht, die durch Dehnung aktiviert wurden.

7. Verbundstoff nach einem der vorhergehenden Ansprüche, wobei der Verbundstoff einen zweilagigen Aufbau aufweist.

8. Verbundstoff nach einem der Ansprüche 1 - 6, wobei der Verbundstoff einen dreilagigen Aufbau aufweist.

9. Verbundstoff nach einem der vorhergehenden Ansprüche, wobei der gesamte Verbundstoff durch Dehnung aktiviert wurde.

10. Verbundstoff nach einem der vorhergehenden Ansprüche, wobei die nicht-elastische Folie aus einem Polymer wie Ethylen, Propylen, Butylen, Penten, Hexen, Hepten oder Octen gebildet ist, wie auch aus Copolymeren, Terpolymeren oder Mischungen dieser, Ethylen-Vinylacetat (EVA), Ethylen-Ethylacrylat (EEA), Ethylen-Acrylsäure (EAA), Ethylen-Methylacrylat (EMA), Ethylen-Butylacrylat, Polyurethan, Poly(ether)ester und Polyamidether-Blockcopolymeren oder einer beliebigen Kombination dieser.

11. Verbundstoff nach einem der vorhergehenden Ansprüche, wobei die elastische Vliesschicht aus Bikomponentenfasern gebildet ist, wobei die Bikomponentenfasern eine innere erste Komponente und eine äußere zweite Komponente umfassen, wobei die zweite Komponente weniger elastisch als die erste Komponente ist, wobei die erste Komponente ein thermoplastisches Elastomer ist, wobei die zweite Komponente zwischen etwa 1 und etwa 20 Prozent der Bikomponentenfasern ausmacht.

12. Verbundstoff nach einem der vorhergehenden Ansprüche, wobei die elastische Vliesschicht aus Bikomponentenfasern gebildet ist, wobei die Bikomponentenfasern eine innere erste Komponente und eine äußere zweite Komponente umfassen, wobei die zweite Komponente weniger elastisch als die erste Komponente ist, wobei die erste Komponente ein thermoplastisches Elastomer ist, wobei die erste Komponente aus einem Polyurethan, einem Block-Copolyester, einem Block-Copolyamid, einem styrolischen Blockcopolymer, einem Polyolefin-Elastomer oder Kombinationen dieser gebildet ist.

13. Verbundstoff nach einem der vorhergehenden Ansprüche, wobei die atmungsaktive, nicht-elastische Folie eine mehrlagige Folie, eine monolithische Folie, eine Gießfolie ist.

14. Verfahren zur Herstellung eines elastischen mehrlagigen Verbundstoffes, umfassend: Ausbilden eines Verbundstoffes aus mindestens einer atmungsaktiven, nicht-elastischen Folienschicht und mindestens einer elastischen Vliesschicht, wobei die elastische Vliesschicht aus Bikomponentenfasern gebildet wird, wobei die Bikomponentenfasern, die eine Kern/Mantel-, eine mehrlappige oder eine spitz mehrlappige Struktur aufweisen, eine innere erste Komponente und eine äußere zweite Komponente umfassen, wobei die zweite Komponente weniger elastisch als die erste Komponente ist, wobei die erste Komponente ein thermoplastisches Elastomer ist, wobei die erste Komponente mindestens 50 % der Fasern ausmacht und wobei die zweite Komponente Polyethylen, Polypropylen oder eine Mischung aus Polyethylen und Polypropylen ist.

15. Verfahren nach Anspruch 14, wobei das Laminat durch Thermopunkt-Verbindung gebildet wird.

16. Verfahren nach Anspruch 14, wobei die Schichten durch Extrusionslaminierung gebildet werden.

17. Verfahren nach Anspruch 14, wobei sich Klebstoff zwischen der atmungsaktiven, nicht-elastischen Folienschicht und der elastischen Vliesschicht befindet.

18. Verfahren nach einem der Ansprüche 14 - 17, wobei das elastische Vlies ein Spinnvlies ist.

19. Verfahren nach einem der Ansprüche 14 - 18, wobei die Vliesschicht aus Bikomponentenfasern besteht, die nicht aktiviert wurden.

20. Verfahren nach einem der Ansprüche 14 - 19, wobei die Vliesschicht aus Bikomponentenfasern besteht, die durch Dehnung aktiviert wurden.

21. Verfahren nach einem der Ansprüche 14 - 20, wobei der Verbundstoff einen zweilagigen Aufbau aufweist, nicht eingeschlossen eine optionale Haftschicht.

22. Verfahren nach einem der Ansprüche 14 - 21, wobei der Verbundstoff einen dreilagigen Aufbau aufweist, nicht eingeschlossen zwei optionale Haftschichten.

23. Verfahren nach einem der Ansprüche 14 - 22, wobei der gesamte Verbundstoff durch Dehnung aktiviert wird.

24. Verfahren nach einem der Ansprüche 14 - 23, wobei die Laminierung durch Schmelzklebelaminierung erfolgt.

25. Verfahren nach einem der Ansprüche 14 - 24, wobei die Laminierung durch Extrusionslaminierung erfolgt.

26. Verfahren nach einem der Ansprüche 14 - 25, wobei die Laminierung an einer Vielzahl von beabstandeten Punkten durch Thermopunkt-Verbindung erfolgt.

27. Verbundstoff nach einem der Ansprüche 14 - 26, wobei die Zugfestigkeit der elastischen Vliesschicht geringer ist als die Zugfestigkeit der nicht-elastischen Folie.

28. Verbundstoff nach einem der Ansprüche 14 - 27, wobei die nicht-elastische Folie aus einem Polymer aus Ethylen, Propylen, Butylen, Penten, Hexen, Hepten oder Octen gebildet wird, wie auch aus Copolymeren, Terpolymeren, oder Mischungen dieser, Ethylen-Vinylacetat (EVA), Ethylen-Ethylacrylat (EEA), Ethylen-Acrylsäure (EAA), Ethylen-Methylacrylat (EMA), Ethylen-Butylacrylat, Polyurethan, Poly(ether)ester und Polyamidether-Blockcopolymeren, oder einer beliebigen Kombination dieser.

29. Verbundstoff nach einem der Ansprüche 14 - 27, wobei die elastische Vliesschicht aus Bikomponentenfasern gebildet ist, wobei die Bikomponentenfasern eine innere erste Komponente und eine äußere zweite Komponente umfassen, wobei die zweite Komponente weniger elastisch als die erste Komponente ist, wobei die erste Komponente ein thermoplastisches Elastomer ist, wobei die zweite Komponente zwischen etwa 1 und etwa 20 Prozent der Bikomponentenfasern ausmacht.

30. Verbundstoff nach einem der Ansprüche 14 - 29, wobei die elastische Vliesschicht aus Bikomponentenfasern gebildet ist, wobei die Bikomponentenfasern eine innere erste Komponente und eine äußere zweite Komponente umfassen, wobei die zweite Komponente weniger elastisch als die erste Komponente ist, wobei die erste Komponente ein thermoplastisches Elastomer ist, wobei die erste Komponente aus einem Polyurethan, einem Block-Copolyester einem Block-Copolyamid, einem styrolischen Blockcopolymer, einem Polyolefin-Elastomer oder Kombinationen dieser gebildet ist.

## Revendications

1. Composite multicouche élastique, comprenant une couche de film non élastique respirable adjacente à une couche non-tissée élastique, dans lequel la couche non-tissée élastique est formée de fibres à deux composants, dans lequel les fibres à deux composants, ayant une structure gaine/coeur, multilobée, ou multilobée rapportée, comprennent un premier composant intérieur et un deuxième composant extérieur, dans lequel le deuxième composant est moins élastique que le premier composant, dans lequel le premier composant est un élastomère thermoplastique, dans lequel le premier composant constitue au moins 50 % des fibres, et dans lequel le deuxième composant est le polyéthylène, le polypropylène, ou un mélange de polyéthylène et de polypropylène.

2. Composite selon la revendication 1, contenant un adhésif entre la couche de film non élastique respirable et la couche non-tissée élastique.

3. Composite selon l'une quelconque des revendications précédentes, dans lequel les couches adhèrent l'une à l'autre en une multiplicité de points, et dans lequel ces points de collage sont formés via chaleur et pression.

4. Composite selon l'une quelconque des revendications précédentes, dans lequel la couche non-tissée élastique est une étoffe filée-liée.

5. Composite selon l'une quelconque des revendications précédentes, dans lequel la couche non-tissée est composée de fibres à deux composants qui n'ont pas été activées.

6. Composite selon l'une quelconque des revendications précédentes, dans lequel la couche non-tissée est composée de fibres à deux composants qui ont été activées par étirage.

7. Composite selon l'une quelconque des revendications précédentes, lequel composite a une structure bicouche.

8. Composite selon l'une quelconque des revendications 1 à 6, dans lequel le composite a une structure tricouche.

9. Composite selon l'une quelconque des revendications précédentes, dans lequel la totalité du composite est activé par étirage.

10. Composite selon l'une quelconque des revendications précédentes, dans lequel le film non élastique est formé d'un polymère d'éthylène, propylène, butylène, pentène, hexène, heptène, et octène, ainsi que leurs copolymères, terpolymères, et mélanges, de copolymères séquencés d'éthylène/acétate de vinyle (EVA), d'éthylène/acrylate d'éthyle (EEA), d'éthylène/acide acrylique (EAA), d'éthylène/acrylate de méthyle (EMA), d'éthylène/acrylate de butyle, de polyuréthane, de poly(éther-ester) et de poly(amide-éther), et de l'une quelconque de leurs combinaisons.

11. Composite selon l'une quelconque des revendications précédentes, dans lequel la couche non-tissée élastique est formée de fibres à deux composants, dans lequel les fibres à deux composants comprennent un premier composant intérieur et un deuxième composant extérieur, dans lequel le deuxième composant est moins élastique que le premier composant, dans lequel le premier composant est un élastomère thermoplastique, dans lequel le deuxième composant est présent dans les fibres à deux composants à raison d'environ 1 à environ 20 %.

12. Composite selon l'une quelconque des revendications précédentes, dans lequel la couche non-tissée élastique est formée de fibres à deux composants, dans lequel les fibres à deux composants comprennent un premier composant intérieur et un deuxième composant extérieur, dans lequel le deuxième composant est moins élastique que le premier composant, dans lequel le premier composant est un élastomère thermoplastique, dans lequel le premier composant est formé à partir d'un polyuréthane, d'un copolyester séquencé, d'un copolyamide séquencé, d'un polymère séquencé styrénique, d'un élastomère de polyoléfine, et de leurs combinaisons.

13. Composite selon l'une quelconque des revendications précédentes, dans lequel le film non élastique respirable est un film multicouche, un film monolithique, un film coulé.

14. Procédé pour fabriquer un composite multicouche élastique, comprenant : la formation d'un composite d'au moins une couche de film non élastique respirable et d'au moins une couche non-tissée élastique, dans lequel la couche non-tissée élastique est formée de fibres à deux composants, dans lequel les fibres à deux composants, ayant une structure gaine/coeur, multilobée, ou multilobée rapportée, comprennent un premier composant intérieur et un deuxième composant extérieur, dans lequel le deuxième composant est moins élastique que le premier composant, dans lequel le premier composant est un élastomère thermoplastique, dans lequel le premier composant constitue au moins 50 % des fibres, et dans lequel le deuxième composant est le polyéthylène, le polypropylène, ou un mélange de polyéthylène et de polypropylène.

15. Procédé selon la revendication 14, dans lequel le stratifié est formé par utilisation d'un collage thermique par points.

16. Procédé selon la revendication 14, dans lequel les couches sont formées par utilisation d'une stratification avec extrusion.

17. Procédé selon la revendication 14, dans lequel un adhésif se trouve entre la couche de film non élastique respirable et la couche non-tissée élastique.

18. Procédé selon l'une quelconque des revendications 14 à 17, dans lequel le non-tissé élastique est un non-tissé filélié.

19. Procédé selon l'une quelconque des revendications 14 à 18, dans lequel la couche non-tissée est composée de fibres à deux composants qui n'ont pas été activées.

20. Procédé selon l'une quelconque des revendications 14 à 19, dans lequel la couche non-tissée est composée de fibres à deux composants qui ont été activées par étirage.

21. Procédé selon l'une quelconque des revendications 14 à 20, dans lequel le composite a une structure bicouche, si l'on excepte une couche adhésive facultative.

22. Procédé selon l'une quelconque des revendications 14 à 21, dans lequel le composite a une structure tricouche, si l'on excepte deux couches adhésives facultatives.

23. Procédé selon l'une quelconque des revendications 14 à 22, dans lequel tout le composite est activé par étirage.

24. Procédé selon l'une quelconque des revendications 14 à 23, dans lequel la stratification est effectuée par utilisation d'une stratification avec un adhésif à l'état fondu.

25. Procédé selon l'une quelconque des revendications 14 à 24, dans lequel la stratification est effectuée par utilisation d'une stratification avec extrusion.

26. Procédé selon l'une quelconque des revendications 14 à 25, dans lequel la stratification est effectuée en une pluralité de points mutuellement espacés par utilisation d'un collage thermique par points.

27. Procédé selon l'une quelconque des revendications 14 à 26, dans lequel la couche non-tissée élastique a une résistance à la traction inférieure à la résistance à la traction du film non élastique.

28. Procédé selon l'une quelconque des revendications 14 à 27, dans lequel le film non élastique est formé d'un polymère d'éthylène, propylène, butylène, pentène, hexène, heptène, et octène, ainsi que leurs copolymères, terpolymères, et mélanges, de copolymères séquencés d'éthylène/acétate de vinyle (EVA), d'éthylène/acrylate d'éthyle (EEA), d'éthylène/acide acrylique (EAA), d'éthylène/acrylate de méthyle (EMA), d'éthylène/acrylate de butyle, de polyuréthane, de poly(éther-ester) et de poly(amide-éther), et de l'une quelconque de leurs combinaisons.

29. Procédé selon l'une quelconque des revendications 14 à 27, dans lequel la couche non-tissée élastique est formée de fibres à deux composants, dans lequel les fibres à deux composants comprennent un premier composant intérieur et un deuxième composant extérieur, dans lequel le deuxième composant est moins élastique que le premier composant, dans lequel le premier composant est un élastomère thermoplastique, dans lequel le deuxième composant est présent dans les fibres à deux composants à raison d'environ 1 à environ 20 %.

30. Procédé selon l'une quelconque des revendications 14 à 29, dans lequel la couche non-tissée élastique est formée de fibres à deux composants, dans lequel les fibres à deux composants comprennent un premier composant intérieur et un deuxième composant extérieur, dans lequel le deuxième composant est moins élastique que le premier composant, dans lequel le premier composant est un élastomère thermoplastique, dans lequel le premier composant est formé à partir d'un polyuréthane, d'un copolyester séquencé, d'un copolyamide séquencé, d'un polymère séquencé styrénique, d'un élastomère de polyoléfine, et de leurs combinaisons.
